# EUROPEAN PATENT APPLICATION

(11) **EP 1 949 847 A1**
(43) Date of publication of application: **30.07.2008**
(21) Application number: 07001733.0
(22) Date of filing: 26.01.2007
(51) Int. Cl.: A61B 1/05, A61B 5/00

(54) **Memory-type two-section endoscopic system**

(71) Applicant: Chung Shan Institute of Science and Technology, Longtan Township T'ao yuan (TW)
(72) Inventor: Wu, Hsien-Ming, Longtan Township Taoyuan County 325 (TW); Huang, Ker-Jer, Longtan Township Taoyuan County 325 (TW); Chen, Yen-Chung, Longtan Township Taoyuan County 325 (TW)
(74) Representative: Kador & Partner

(57) **Abstract**

The present invention provides a memory-type two-section endoscopic system, which includes an endoscope that comprises a light-emitting device, an image-capturing device, a storage module, and a transmission module adapted in a shell. A disconnectable cable connects to the shell, and can depart form the shell. When the endoscope examines the esophagus and the stomach, the disconnectable cable can be used to control the endoscope for obtaining image data of the esophagus and the stomach for the image-capturing device. Then the image data is stored in the storage module or transmitted to the transmission module. When the endoscope examines the intestines, the shell will depart from the disconnectable cable and the endoscope will continue examinations of the intestines without wire control. The image data obtained by examining the intestines is stored to the storage module. Thereby, the esophagus, the stomach, and the intestines can be examined at a time, and the efficiency of an endoscope is enhanced.

## Description

### FIELD OF THE INVENTION

The present invention relates generally to an endoscopic system, and particularly to a memory-type two-section endoscopic system.

### BACKGROUND OF THE INVENTION

An endoscopic system is used extensively in modem surgical examination of clinical medicine for acquiring medical images of internal organs and tissues of a human body, or performing small-scale surgical operations. The endoscopic system according to the prior art is used for going deep into hollow organs of a human body, such as the stomach, the large intestine, and the trachea, for obtaining organ images, and thereby the type of the nidus in the hollow organs of a human body and the level of development thereof can be judged. The endoscopic system according to the prior art adopts a stranded optical fiber system to transmit image data obtained by an optical-fiber-type endoscopic system in a human body. The stranded optical fiber system uses a stranded optical fiber cable to guide light of a light source to an endoscope in the human body. After the light of the light source illuminates organs and tissues in the human body via the endoscope, the endoscope receives reflected light from the organs and tissues, and transmits the reflected light to an image-sensing device for sensing image data. Then, the image data sensed by the image-sensing device is displayed on a display after image processing.

Nevertheless, normal stranded optical fiber cable is composed of a plurality of optical fibers. Thereby, images with sufficient pixels can be transmitted by the plurality of optical fibers and organs and tissues can be displayed. A major problem of current optical-fiber-type endoscope is that the diameter of the soft tube connecting the optical-fiber-type endoscope is too thick because of the optical fibers, which causes patients taking endoscopic examinations feel uncomfortable by swallowing the soft tube of the endoscope. Hence, the patients will fear of taking endoscopic examinations. In addition, because the optical-fiber-type endoscope is costly, it has to be reused. Thereby, patients taking endoscopic examinations tend to be cross-infected due to improper sterilization of the reused endoscopes. Moreover, present optical-fiber-type endoscope can only examine the esophagus, the stomach, and the large intestine. For the small intestine with total length over six meters, optical-fiber-type endoscope can enter the first 90-centimeter portion of the small intestine. Thereby, other portions thereof cannot be examined.

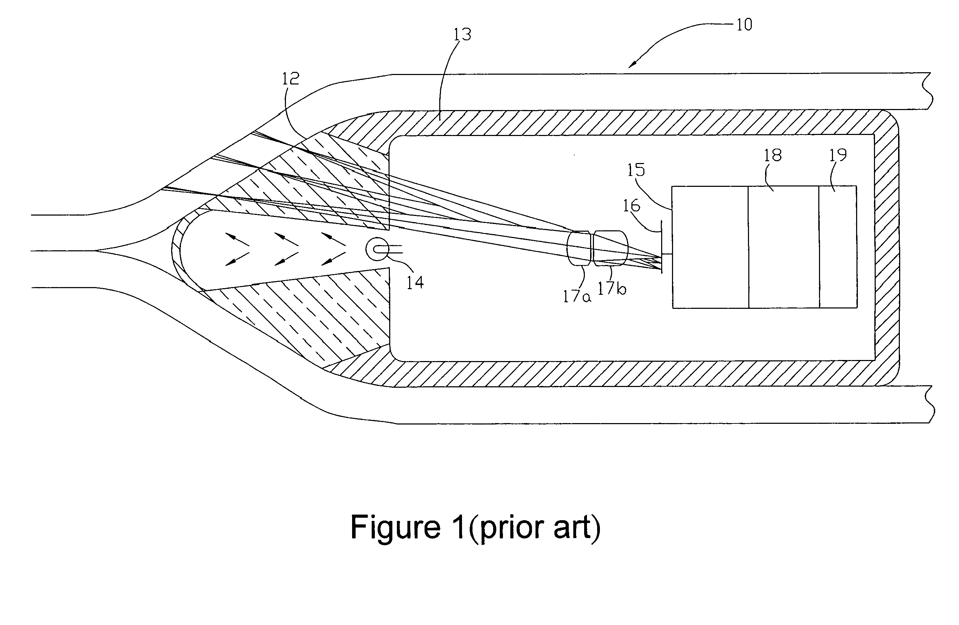

In order to solve the problem of examinations on the small intestine, a capsule-type endoscopic system with wireless transmission is developed. Figure 1 shows a structural schematic diagram of a capsule-type endoscope according to the prior art. The capsule-type endoscope according to the prior art is published in the US patent No. 5,604,531 entitled "In vivo video camera system". The capsule-type endoscope 10 uses wireless transmission interface to transmit image data obtained by the capsule-type endoscope 10 for examining inner wall of the small intestine. The shell of the capsule-type endoscope 10 is comprised of a transparent optical front cover 12 and an opaque capsule housing 13. The capsule-type endoscope 10 comprises a light-emitting diode 14, a charge-coupled device driver 15, a charge-coupled device 16, an imaging optical front lens 17a, an imaging optical rear lens 17b, a wireless transmitter 18, and a power supply 19. The power supply supplies power to the light-emitting diode 14, the charge-coupled device driver 15, the charge-coupled device 16, and the wireless transmitter 18. (The assignee of this invention is State of Israel, Ministry of Defense, Armament Development Authority (Rafael, IL).)

When a patient swallows the wireless capsule-type endoscope 10, the inner wall of human small intestine will be close next to the transparent optical front cover 12. The light-emitting diode 14 illuminates the inner wall of the small intestine through the transparent optical front cover 12, and the light reflected from the inner wall of the small intestine passes through the transparent optical front cover 12, the imaging optical front lens 17a, and the imaging optical rear lens 17b. Then, the charge-coupled device 16 receives the light and produces image data, which is transmitted by the charge-coupled device driver 15 to the wireless transmitter 18. The wireless transmitter 18 transmits a radio-frequency signal corresponding to the image data to an antenna outside the human body for making the antenna receive the radio-frequency signal corresponding to the image data to a receiver. After signal processing on the radio-frequency signal, the result is stored in a storage unit or displayed on a display for providing a doctor to study the patient's condition. The capsule-type endoscope is very effective for obtaining medical images of the small intestine. However, because the capsule-type endoscope cannot control the direction of the lens in the esophagus and the stomach, it is not suitable for examining them.

The capsule-type endoscopes disclosed in the Taiwan Patent No. M242178 "Capsule Endoscopes with Adjusting Focus Function" and the Taiwan Patent No. M242179 "Capsule Endoscopes with Rotatable Image Sensing Module" improve image extraction range and quality of endoscopes, but they cannot control the position of the capsule-type endoscopes in the esophagus and the stomach. In addition, the number of patients with small intestine diseases is far smaller than that of patients with stomach diseases. Thereby, the applications of capsule-type endoscopes are quite limited.

Accordingly, a novel memory-type two-section endoscopic system is provided to improve the drawbacks of traditional endoscopic systems. In addition, the position of the endoscope in the esophagus and the stomach can be adjusted, and the endoscope can be scrapped after use. Thereby, the problems described above can be solved.

### SUMMARY

The objective of the present invention is to provide a memory-type two-section endoscopic system, which adopts a disconnectable cable connecting to a shell of an endoscope for controlling the endoscope to examine in the esophagus and the stomach. In addition, the disconnectable cable can disconnect from the shell for making the endoscope continue to examine the intestines. Hence, the esophagus, the stomach, and the intestines can be examined at a time.

Another objective of the present invention is to provide a memory-type two-section endoscopic system, which adopts a disconnectable cable connecting to a shell of an endoscope for providing power and signals to the endoscope. In addition, when the disconnectable cable disconnects from the shell, the endoscope is driven to use the power supplied by a power supply in the shell.

A further objective of the present invention is to provide a memory-type two-section endoscopic system, which includes a storage module in a shell of an endoscope for storing image data produced by the endoscope.

The present invention is a memory-type two-section endoscopic system, which comprises a disconnectable cable and a memory-type two-section endoscope, which is a capsule-type endoscope, and comprises a light-emitting device, a image-capturing device, a storage module, and a transmission module housed in a shell. The shell connects to the disconnectable cable, which, in addition to providing transmission of power and signals, can be controlled by an external button to disconnect from the shell of memory-type two-section endoscope. When a patient swallows the endoscope, the light-emitting device in the shell of the endoscope will illuminate organs and tissues of the esophagus and the stomach. After image data is obtained by the image-capturing device, the image data is transmitted outside through the disconnectable cable by the transmission module, and then is displayed real-timely or stored separately. Besides, the endoscope further comprises a power supply, which is activated to supply power to the endoscope after the endoscope disconnects from the disconnectable cable when stomach examination is completed. Thereby, the image-capturing device of the endoscope can continue to obtain image data in the small intestine, and the storage module of the endoscope is activated at the same time for storing the image data acquired by the image-capturing device. When the endoscope is expelled from the human body, a micro-connector is used to connect to the storage module of the endoscope for retrieving the image data. Accordingly, the endoscopic system according to the present invention can finish examining the esophagus, the stomach, and the intestines of the digestive system at a time.

### BRIEF DESCRIPTION OF THE DRAWINGS

Figure 1 shows a schematic diagram of a capsule-type endoscope according to the prior art;
Figure 2 shows a schematic diagram of a memory-type two-section endoscope according to a preferred embodiment of the present invention;
Figure 3A shows a structural schematic diagram of a disconnectable structure according to a preferred embodiment of the present invention;
Figure 3B shows a cross-sectional view of steel balls of a disconnectable structure according to a preferred embodiment of the present invention;
Figure 3C shows a cross-sectional view of a flexible tube of a disconnectable structure according to a preferred embodiment of the present invention; and
Figure 4 shows a structural schematic diagram of a disconnected disconnectable structure according to a preferred embodiment of the present invention.

### DETAILED DESCRIPTION

In order to make the structure and characteristics as well as the effectiveness of the present invention to be further understood and recognized, the detailed description of the present invention is provided as follows along with preferred embodiments and accompanying figures.

The memory-type two-section endoscopic system according to the present invention comprises a memory-type two-section endoscope and a disconnectable cable. The memory-type two-section endoscope includes a shell, a light-emitting device, an image-capturing device, a storage module, and a transmission module. The shell has a transparent hood, which connects to the shell. The light-emitting device is adapted in the shell, and is used for providing a light source. The light of the light source illuminates a target through the transparent hood. The image-capturing device is used for receiving the reflected light of the target, and converting it to image data. The storage module couples to the image-capturing device, and is used for storing the image data. The transmission module receives a plurality of control signals via the disconnectable cable for the image-capturing device and the storage module, and for transmitting the image data as well. The disconnectable cable has a disconnectable structure, which is connected at the rear end of the shell. Besides, the shell further has a power supply, which couples to the light-emitting device, the image-capturing device, and the storage module. When the endoscope departs from wire control, the power supply will be activated to supply power to the light-emitting device, the image-capturing device, and the storage module.

Thereby, when the memory-type two-section endoscope is used to examine the inner walls of a patient's esophagus and stomach, high luminance and high-speed medical image data will be provided through the disconnectable cable via the transmission module. In addition, when the memory-type two-section endoscope is used to examine the inner wall of a patient's small intestine, the storage module will be used to store image data. After the memory-type two-section endoscope is expelled from the human body, the stored image data will be retrieved.

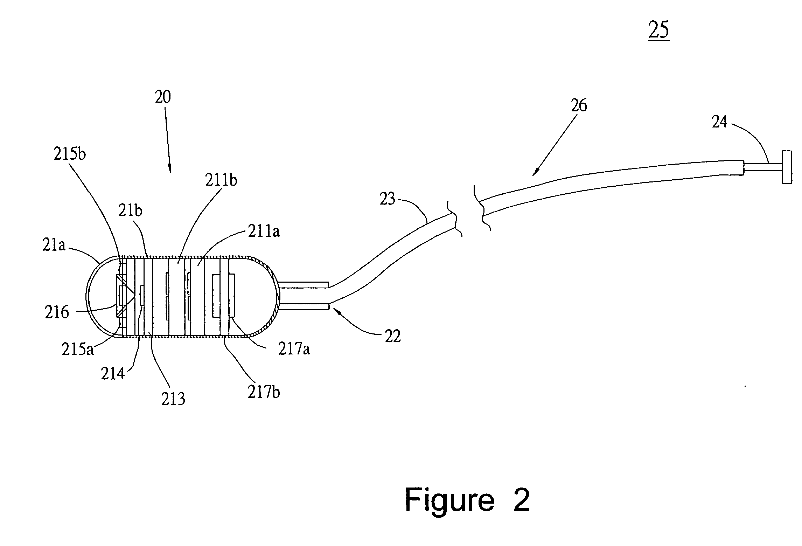

Figure 2 shows a schematic diagram of a memory-type two-section endoscope according to a preferred embodiment of the present invention. The memory-type two-section endoscope system 25 according to the present invention includes an endoscope 20 and a disconnectable cable 26. The endoscope 20 includes a shell, an image-capturing device, and a data storage control device. The shell is made of a plastic material compatible with human body, and the diameter thereof must be smaller than 11 millimeters. The shell includes a transparent hood 21 a and a capsule shell 21b. The image-capturing device has a driving circuit for image sensing 213, an image sensor 214, a light-emitting device 215a, a light-emitting device 215b, and an imaging lens set 216. In the present embodiment, the light-emitting device 215a and the light-emitting device 215b are adapted at the front end of the endoscope 20. The imaging lens set 216 is adapted between the light-emitting device 215a and the light-emitting device 215b. The light-emitting device 215a and the light-emitting device 215b are light-emitting diodes. In addition, the image sensor 214 is adapted behind light-emitting device 215a, the light-emitting device 215b, and the imaging lens set 216.

The storage module 217a has a plurality of dynamic random access memories (DRAMs), a plurality of nanometer-scale non-volatile memories, or a combination of said two memory types, for storing image data produced by the image sensor 214. When the light emitted by the light-emitting device 215a and the light-emitting device 215b passes through the transparent hood 21a and illuminates on organs and tissues of a human body, the reflected light from the organs and tissues will pass through the transparent hood 21 a and the imaging lens set 216, and form images on the image sensor 214. The image sensor 214 converts the optical images formed thereon to image data via the driving circuit for image sensing 213. The image data can be transmitted through two paths as shown below.

First path: When the endoscope 20 is in the esophagus and the stomach of a human body, the disconnectable cable 26 can be used to transmit power and signals. The image data will be transmitted from the transmission module 217b through a disconnect module 22 and a flexible soft tube 23 to a transmission control device (not shown in the figure). Then, the image data is stored or displayed on a display after image processing.

Second path: When the endoscope 20 performs examinations in the intestines of a human body, the shell 21b will depart from the disconnectable cable and the endoscope 20 will operate without wire control. Then the storage module 217a stores image data produced by the image sensor 214 and the driving circuit for image sensing 213. After the endoscope 20 is expelled from the human body, the image data stored in the storage module 217a is retrieved.

The endoscope 20 further installs a battery 211 a and a battery 211b. When the endoscope 20 performs examinations in the intestines of a human body and thereby is driven to depart from wire control, the internal battery 211a and battery 211b will supply power to the light-emitting devices 215a, 215b, the image sensor 214, and the storage module 217a. Because the batteries 211a, 211b are needed only when the endoscope 20 breaks from the disconnectable cable 26 and continues examinations in the intestines, the power demand on the batteries 211a, 211b is relatively small. Thereby, the batteries 211a, 211b can be made smaller, which is easier for a patient to swallow the endoscope 20. Moreover, the storage module 217a further has a control chip, which controls the storage module 217a to access data. When the endoscope 20 departs from wire control, the control chip will use the power supplied by the batteries 211a, 211b, and control the storage module 217a to access data.

The disconnectable cable 26 has a disconnectable structure, which disconnects by mechanical means or by burning of electrical currents. The disconnectable structure according to the present embodiment adopts disconnection by mechanical means. The disconnectable structure includes a disconnect module 22, a flexible soft tube 23, which is compatible with a human body, and a disconnect button 24. The disconnect module 22 is adapted between the endoscope 20 and the disconnectable cable 26. When the endoscope 20 examines the esophagus and the stomach of a human body, the endoscope 20 is wire-controlled through the disconnectable cable 26. The disconnectable cable 26 power lines and signal lines, which are electrically connected with the internal circuits of the endoscope 20 through the external flexible soft tube 23 and the disconnect module 22.

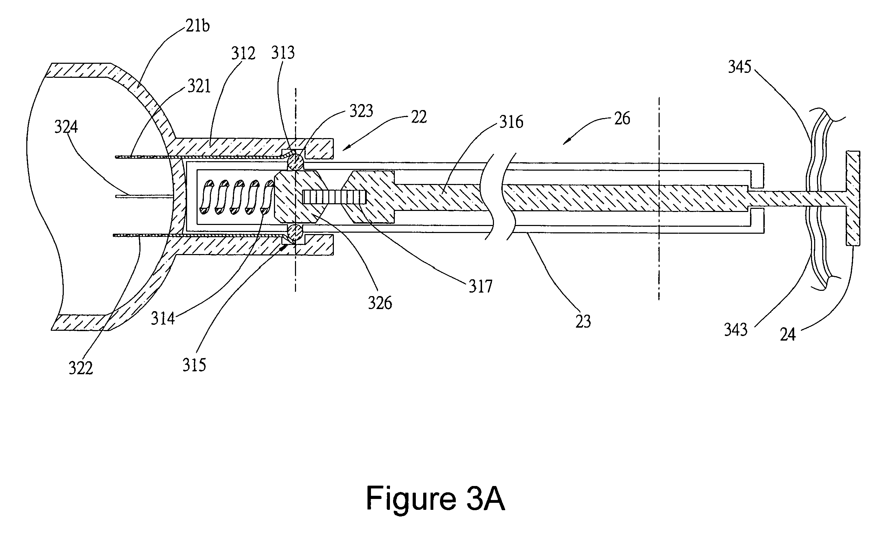

Figure 3A shows a structural schematic diagram of a disconnectable structure according to a preferred embodiment of the present invention. The flexible soft tube 23 of the disconnectable cable 26 connects a disconnect module 22. The flexible soft tube 23 further connects to a disconnect shell 312 via the disconnect module 22. The disconnect shell 312 is a shell extended from the capsule shell 21b. In addition, the disconnect shell 312 has a plurality of leaf springs 313, and the disconnect module 22 has a plurality of steel-ball grooves 315, a plurality of steel balls 323, and a pushing block 326. When the disconnect module 22 connects to the disconnect shell 312, the steel balls 323 will protrude the surface of the flexible soft tube 23, and thereby pushing the leaf springs into the steel-ball grooves 315 of the disconnect shell 312. Hence, the flexible soft tube 23 connects with the disconnect shell 312 and will not loose. Accordingly, the steel balls 323 contact tightly with the leaf springs 313, which is connected with a signal line 321 of the endoscope 20. Thereby, the image data obtained by the endoscope 20 can be transmitted to the external transmission control device through the signal line 312 for storing or displaying. The endoscope 20 couples to external power supply via a power line 324 if the flexible soft tube 23 for supplying external power to the endoscope 20 through the power line 324.

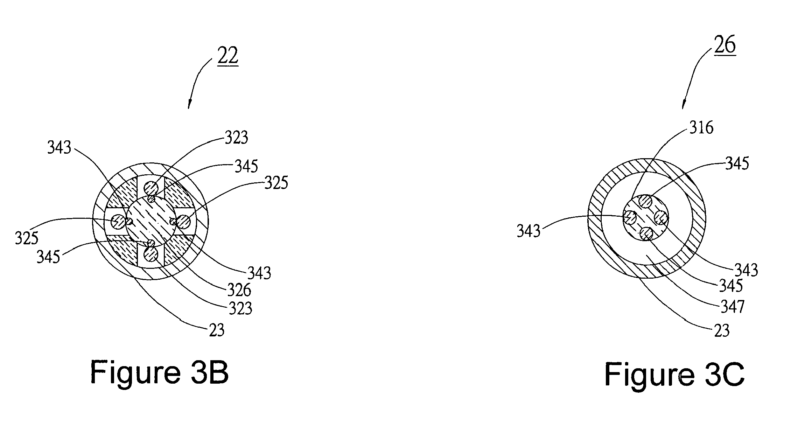

Figure 3B shows a cross-sectional view of the steel balls of the disconnectable structure according to Figure 3A. The steel balls include first steel balls 323 and second steel balls 325. The first steel balls 323 connect with signal lines 345, while the second steel balls 325 connect with power lines 343. The power lines 343 and the signal lines 345 are embedded in the pushing block 326. An axle center 316 is made of firm plastic materials. If the first steel balls 323 and the second steel balls 325 are located in the steel-ball grooves 315, all devices of the disconnect module 22 are located in the disconnect shell 312 such that the flexible soft tube 23 connects to the disconnect shell 312 via the disconnect module 22. Figure 3C shows a cross-sectional view of the flexible soft tube 23 according to Figure 3A. The power lines 343 and the signal lines 345 are inlaid in the axle center 316. A gap 347 exists between the outer surface of the axle center 316 and the inner side of the flexible soft tube 23. When the endoscope 20 operates in the esophagus and the stomach of a human body, external power will be supplied through the power lines 343 contained in the flexible soft tube 23 of the disconnectable cable 26 to the light-emitting devices 215a, 215b, the image-capturing device 214, and the storage module 217a.

The power of the endoscope 20 according to the present invention is not limited when it is connected to the external power supply via the power lines 343 of the disconnectable cable. Thereby, the light-emitting devices 215a, 215b can provide light sources with high luminance in terms of external power supplies. Furthermore, image data can be transmitted via the signal lines 345 contained in the disconnectable cable 26, and the image quality, as well as frames per second of the endoscope 20, is comparable to traditional endoscopy of stomachs. The image data is transmitted by a plurality of signal lines 345 in the disconnectable cable 26. In addition, the outer layer of the disconnectable cable 26 is a flexible soft tube 23, the bending behavior of which is controllable. However, the overall diameter of the disconnectable cable is still smaller than the optical-fiber strands of an optical-fiber-type endoscope. Moreover, the bending behavior of the flexible soft tube 23 of the disconnectable cable 26 is superior with extreme small radius of curvature. On the contrary, the radius of curvature of an optical-fiber-type endoscope is substantially large due to the poor bending behavior of optical fibers. In order to make an optical-fiber-type endoscope have better bending behavior, substantial costs and time have to be paid to improve equipments and configurations.

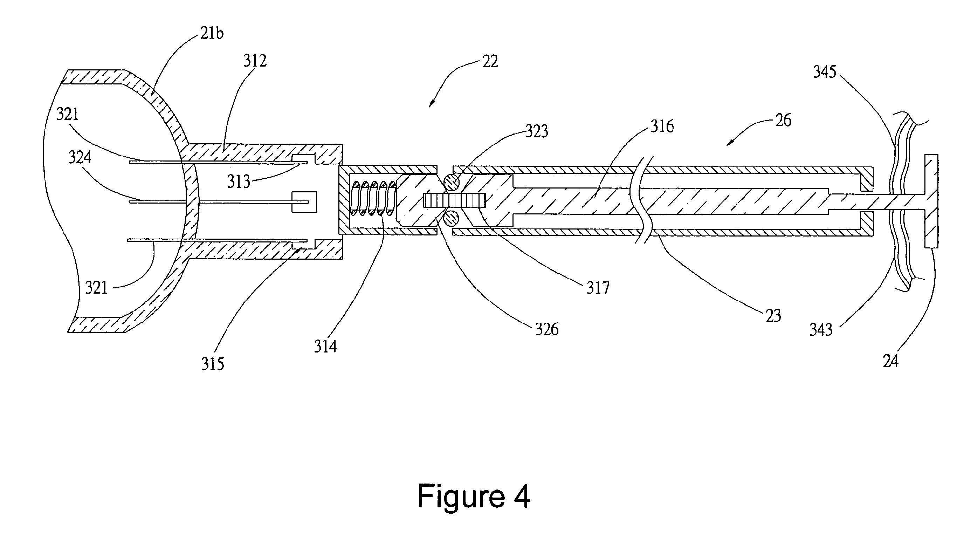

When the endoscope 20 completes examination of stomach, the physician can push the disconnect button 24, which will push the axle center 316, which, in turn, will push the pushing block 326 to move forward and thereby compress a spring 314. At the same time, the first steel balls 323 and the second steel balls 325 will be attracted by a high-intensity magnet 317 and will be trapped to the steel-ball grooves 315. At this moment, the disconnectable cable 326 does not buckle the disconnect shell 312 by the first and the second steel balls 323, 325. Thereby, the flexible soft tube 23 departs from the disconnect shell 312 smoothly, as shown in Figure 4.

Figure 4 shows the situation after the flexible soft tube 23 is separated from the endoscope 20. At this time, the disconnect button 24 is pushed, and the axle center 316 as well as the pushing block 326 are moved forward. In addition, the spring 314 is compressed, the leaf springs 313 resume straight, and the first steel balls 323 are attracted by the high-intensity magnet 317 and thereby have lost connection effect. After disconnection from the disconnectable cable 26, the endoscope 20 enters the intestines. Besides, the circuit of the endoscope 20 further has a switching circuit. When the first steel balls 323 and the second steel balls 325 are attracted by the high-intensity magnet 317, the switching circuit is driven to activate the internal power of the endoscope 20, that is, the batteries 211a, 211b, which supply power in succession to the endoscope 20. Thereby, the endoscope 20 behaves like a normal camera capturing pictures in the intestines. Accordingly, the memory-type two-section endoscope 20 owns the advantages of an optical-fiber-type endoscope of stomachs and a capsule-type endoscope simultaneously. The examinations of the alimentary canal of a digestive system, that is, the esophagus, the stomach, and the intestines, can thereby be achieved thoroughly.

Moreover, the transmission module 217b of the endoscope 20 according to the present invention can transmit image data to the transmission control device via a wireless transmission interface, and receive the control signal transmitted by the transmission control device. With the aid of the batteries 211a, 211b, it can examine the inner walls of the intestines without wire control. The disconnectable cable 26 according to the present invention can further connects to an endoscope with wireless transmission for adjusting positions of the endoscope with wireless transmission in the esophagus and the stomach. It can be used to adjust positions of the endoscope with wireless transmission in the large intestine. The endoscope with wireless transmission is a capsule-type endoscope.

Accordingly, the present invention conforms to the legal requirements owing to its novelty, non-obviousness, and utility. However, the foregoing description is only a preferred embodiment of the present invention, not used to limit the scope and range of the present invention. Those equivalent changes or modifications made according to the shape, structure, feature, or spirit described in the claims of the present invention are included in the appended claims of the present invention.

## Claims

1. A memory-type two-section endoscopic system, comprising:
a shell, having a transparent hood covering the shell;
a light-emitting device, adapted in the shell, and producing a light source illuminating a target through the transparent hood;
an image-capturing device, adapted in the shell, receiving the reflected light from the target and producing image data;
a storage module, adapted in the shell, and storing the image data;
a transmission module, adapted in the shell, connecting with the storage module and the image-capturing device, transmitting the image data and receiving a control signal to control the image-capturing device and the storage module; and
a disconnectable cable, connecting to the shell, and disconnectable from the shell.

2. The memory-type two-section endoscopic system of claim 1, and further comprising a transmission control device, which couples to the disconnectable cable, transmits the control signal to the transmission module via the disconnectable cable and a wireless transmission interface, and receives the image data transmitted by the transmission module via the disconnectable cable and the wireless transmission interface.

3. The memory-type two-section endoscopic system of claim 2, wherein when the disconnectable cable disconnects from the shell, the transmission control device will be driven to transmit the control signal to the transmission module via a wireless transmission interface, and to receive the image data transmitted by the transmission module via the wireless transmission interface.

4. The memory-type two-section endoscopic system of claim 1, wherein when the disconnectable cable disconnects from the shell, the transmission module will be driven to stop transmitting the image data and receiving the control signal, and to store the image data to the storage module.

5. The memory-type two-section endoscopic system of claim 1, and further comprising a transmission control device, which transmits the control signal to the transmission module via a wireless transmission interface, and receives the image data transmitted by the transmission module via the wireless transmission interface.

6. The memory-type two-section endoscopic system of claim 1, and further comprising a disconnect module, which is adapted between the shell and the disconnectable cable, and disconnects the disconnectable cable from the shell.

7. The memory-type two-section endoscopic system of claim 6, wherein the disconnectable cable further has a disconnect button, which connects to the disconnect module, and controls the disconnect module to disconnect from the shell.

8. The memory-type two-section endoscopic system of claim 6, wherein the disconnectable cable further has a current output device, which connects to the disconnect module, and produces a current to drive the disconnect module to disconnect from the shell.

9. The memory-type two-section endoscopic system of claim 1, and further comprising a power supply, which couples power to the image-capturing device, the light-emitting device, and the storage module.

10. The memory-type two-section endoscopic system of claim 9, and when the disconnectable cable disconnects from the shell, the power supply will be driven to supply power to the image-capturing device, the light-emitting device, and the storage device.

11. The memory-type two-section endoscopic system of claim 1, wherein the disconnectable cable includes a plurality of power lines and a plurality of signal lines, the plurality of power lines supplying power to the image-capturing device, the light-emitting device, the storage device, and the transmission module, and the plurality of signal lines transmitting the control signals to the image-capturing device, the storage device, and the transmission module.

12. The memory-type two-section endoscopic system of claim 1, wherein the disconnectable cable includes a flexible soft tube, which covers the disconnectable cable and connects to the shell.

13. The memory-type two-section endoscopic system of claim 1, wherein the shell is a capsule shell.

14. The memory-type two-section endoscopic system of claim 1, wherein the light-emitting device is a light-emitting diode.

15. The memory-type two-section endoscopic system of claim 1, wherein the storage module is a memory module.

16. The memory-type two-section endoscopic system of claim 15, wherein the memory module has a plurality of dynamic random access memories (DRAMs), a plurality of nanometer-scale non-volatile memories, or a combination of said two memory types.

17. The memory-type two-section endoscopic system of claim 15, wherein the memory module further has a control chip controlling data access of the memory module.
